(19) Europäisches Patentamt
European Patent Office
Office européen des brevets

(11) Veröffentlichungsnummer: **0 219 666 B1**

# (12) EUROPÄISCHE PATENTSCHRIFT

(45) Veröffentlichungstag der Patentschrift: **15.05.91**

(51) Int. Cl.[5]: **C07F 7/18**, C07C 35/06, C07D 303/14

(21) Anmeldenummer: **86112318.0**

(22) Anmeldetag: **05.09.86**

## (54) Cyclopentanderivate.

(30) Priorität: **20.09.85 DE 3533576**

(43) Veröffentlichungstag der Anmeldung:
**29.04.87 Patentblatt 87/18**

(45) Bekanntmachung des Hinweises auf die Patenterteilung:
**15.05.91 Patentblatt 91/20**

(84) Benannte Vertragsstaaten:
**AT BE CH DE FR GB IT LI NL SE**

(56) Entgegenhaltungen:
**DE-A- 3 401 542**
**US-A- 4 127 736**

**JOURNAL OF THE AMERICAN CHEMICAL SOCIETY, Band 94, Juni-Juli 1972, Seiten 4343-4345, Washington D.C., US; J.FRIED et al.: "Regiospecific Epoxide Opening with Acetylenic Alanes. An Improved Total Synthesis of E an F Prostaglandins"**

(73) Patentinhaber: **MERCK PATENT GESELLSCHAFT MIT BESCHRÄNKTER HAFTUNG**
**Frankfurter Strasse 250 Postfach 4119**
**W-6100 Darmstadt(DE)**

(72) Erfinder: **Riefling, Bernhard, Dr.**
**Zöllerstrasse 28**
**W-6100 Darmstadt(DE)**
Erfinder: **Witzel, Hans**
**Darmstädterstrasse 44**
**W-6110 Dieburg(DE)**

EP 0 219 666 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Cyclopentanderivate der

Formel I

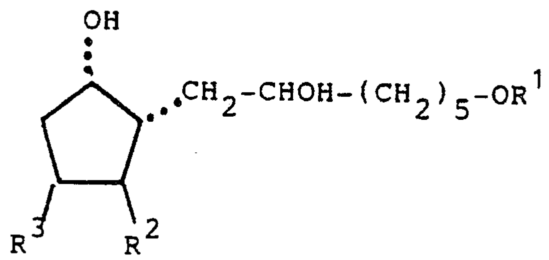

I

worin
$R^1$ H oder eine Hydroxyschutzgruppe und
$R^2$ und $R^3$ zusammen ein O-Atom oder auch zusammen eine Bindung
bedeuten,
sowie ein Verfahren zu ihrer Herstellung, dadurch gekennzeichnet, daß man ein Lactol der Formel II

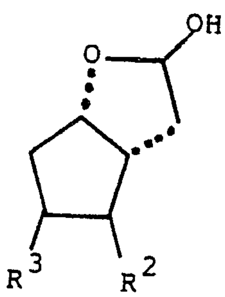

II

worin
$R^2$ und $R^3$ die angegebene Bedeutung haben
mit einer Verbindung der Formel III

$$M-(CH_2)_5-OR^1 \qquad III$$

worin
M MgHal oder Li und
Hal Cl, Br oder J bedeuten und
$R^1$ die angegebene Bedeutung hat,
umsetzt
und daß man gegebenenfalls eine erhaltene Verbindung der Formel I ($R^2$ + $R^3$ = Bindung) durch Behandeln mit einem Oxydationsmittel in eine Verbindung der Formel I ($R^2$ + $R^3$ = O) umwandelt.

Die Verbindungen der Formel I können als Zwischenprodukte bei der Herstellung von Prostaglandinderivaten verwendet werden. Insbesondere ist aus I ($R^2$ + $R^3$ = O) die 6-Oxo-7-(2α,3α-epoxy-5-oxo-lα-cyclopentyl)-heptansäure ("A") erhältlich, aus der z.B. 6,9-Diketo-13-thiaprostaglandine hergestellt werden können (vgl. DE-OS 34 01 542).

Für die Herstellung der Säure "A" ist l.c. eine dreistufige Synthese angegeben, bei der man von 2α-(6-Carboxy-2-cis-hexenyl)-3α,4α-epoxy-cyclopentan-1α-ol ausgeht. Jedoch lassen die Ausbeuten bei dieser Synthese zu wünschen übrig, insbesondere bei Versuchen, sie in den großtechnischen Maßstab zu

übertragen.

Der Erfindung lag die Aufgabe zugrunde, ein neues Verfahren (und neue Zwischenprodukte) zur Herstellung von "A" und verwandten Verbindungen und damit zur Herstellung von Prostaglandinderivaten aufzufinden, das die Nachteile des bekannten Verfahrens nicht oder nur in geringerem Maße aufweist, hohe Ausbeuten liefert und für den großtechnischen Maßstab geeignet ist.

Diese Aufgabe wurde durch die Bereitstellung des neuen Verfahrens und der neuen Zwischenprodukte der Formel I gelöst.

Es wurde gefunden, daß das neue Verfahren, das von leicht zugänglichen Verbindungen (II) ausgeht, bessere Ausbeuten als das bekannte liefert und sich ohne Schwierigkeiten in den großtechnischen Maßstab übertragen läßt.

In den Verbindungen der Formeln I und III kann der Rest $R^1$ eine Hydroxyschutzgruppe bedeuten. Der Ausdruck "Hydroxyschutzgruppe" ist allgemein bekannt und bezeichnet eine Gruppe, die geeignet ist, die Hydroxygruppe vor chemischen Umsetzungen zu schützen (zu blockieren), die aber leicht entfernbar ist, nachdem die gewünschte chemische Reaktion an anderen Stellen des Moleküls durchgeführt worden ist. Natur und Größe der Hydroxyschutzgruppen ist nicht kritisch, da sie nach der gewünschten chemischen Reaktion oder Reaktionsfolge wieder entfernt werden.

Als Hydroxyschutzgruppen eignen sich z.B. unsubstituierte oder substituierte Acylgruppen, Ethergruppen oder Silylethergruppen. Bevorzugte Hydroxyschutzgruppen sind Trialkylsilyl mit insgesamt 3-12 C-Atomen, vorzugsweise Trimethylsilyl, Triethylsilyl, Triisopropylsilyl,tert.-Butyl-dimethylsilyl, Di-tert.-butyl-methylsilyl; Aryldialkylsilyl mit insgesamt 8-18 C-Atomen, vorzugsweise Phenyldimethylsilyl; Tetrahydro-2-pyranyl; Alkoxymethyl mit 2-5 C-Atomen wie Methoxymethyl, Ethoxymethyl oder tert.-Butoxymethyl; Alkylthiomethyl mit 2-5 C-Atomen wie Methylthiomethyl, Ethylthiomethyl oder tert.-Butylthiomethyl; Aryloxymethyl mit 7-11 C-Atomen wie Phenoxymethyl; ferner auch Alkyl mit 1-7 C-Atomen, vorzugsweise tert.-Butyl, Methyl, Ethyl oder Propyl, ferner Isopropyl, Butyl, Isobutyl, sek.-Butyl, Pentyl, Hexyl oder Heptyl; Acyl mit 1-10 C-Atomen, insbesondere Alkanoyl mit 1-6 C-Atomen wie Formyl, Acetyl, Trimethylacetyl, tert.-Butylacetyl; Aroyl mit 7-10 C-Atomen wie Benzoyl; weiterhin Kohlensäureestergruppen wie tert.-Butoxycarbonyl oder Benzoxycarbonyl. Besonders bevorzugt steht $R^1$ für eine Trimethylsilylgruppe.

Die Lactole der Formel II, erhältlich durch Reduktion der entsprechenden Lactone mit Diisobutylaluminiumhydrid, umschließen das bevorzugte 2-Oxa-lßH,5ßH-bicyclo[3,3,0]oct-5-en-3-ol (IIa) und die beiden stereoisomeren 2-Oxa-6,7-epoxy-bicylo[3,3,0]octan-3-ole. Die metallorganischen Verbindungen der Formel III sind aus 5-Hal-pentanolen, erhältlich, die zunächst durch Schutz der OH-Gruppe in Verbindungen der Formel Hal-$(CH_2)_5$-$OR^1$ übergeführt werden können; diese kann man anschließend mit Li oder Mg umsetzen.

Die Umsetzung der Verbindungen der Formel II and III gelingt zweckmäßig in Gegenwart eines inerten Lösungsmittels, z.B. eines Ethers wie Tetrahydrofuran (THF), Diethylether, Diisopropylether, Methyl-tert.-butylether, bei Temperaturen zwischen etwa 0 und 100°, vorzugsweise zwischen 15 und 30°. Zweckmäßig wird die metallorganische Verbindung III in situ hergestellt; anschließend versetzt man mit dem Lactol II oder einer Lösung desselben.

Ein so erhaltenes Cyclopentenderivat I ($R^2$ + $R^3$ = Bindung) kann, falls erwünscht, zum entsprechenden Epoxid I ($R^2$ + $R^3$ = O) oxydiert werden. Als Oxydationsmittel eignen sich insbesondere Peroxide und Hydroperoxide wie m-Chlorperbenzoesäure oder tert.-Butylhydroperoxid. Hydroperoxide werden zweckmäßig in Gegenwart eines Katalysators verwendet, z.B. eines Schwermetallkatalysators wie Molybdänhexacarbonyl, Vanadin(IV)oxid oder einem ihrer Derivate, z.B. Vanadin(IV)oxid-acetylacetonat. Die Oxydation erfolgt zweckmäßig in Gegenwart eines inerten Lösungsmittels, z.B. eines halogenierten Kohlenwasserstoffs wie Dichlormethan, Chloroform, Tetrachlorkohlenstoff, Trichlorethylen, oder eines aromatischen Kohlenwasserstoffs wie Benzol oder Toluol, bei Temperaturen zwischen etwa 0 und etwa 40°, vorzugsweise zwischen I5 und 30°.

Es ist auch möglich, mehrstufig zu oxydieren, z.B. durch Anlagerung von HOBr zum Bromhydrin und anschließende Dehydrobromierung zum Epoxid.

Die erfindungsgemäß erhältlichen Diole der Formel I können zu entsprechenden Diketoverbindungen oxydiert werden. Oxydation von I ($R^1$ = Trimethylsilyl, $R^2$ + $R^3$ = O) mit "Jones-Reagenz" ($CrO_3$ in wässeriger Schwefelsäure) führt z.B. zu der bekannten Säure "A" (l.c.).

"Übliche Aufarbeitung" bedeutet: man extrahiert mit Ethylacetat oder Dichlormethan, wäscht mit Wasser, trocknet die organische Phase und dampft sie ein.

Beispiel

a) Zu einem siedenden Gemisch von 243 g Mg-Spänen, 200 mg Jod und 1 l THF tropft man unter Stickstoff innerhalb 2 Std. eine Lösung von 1795 g 5-Trimethylsilyloxy-pentylbromid (erhältlich aus 5-Brompentanol und Chlortrimethylsilan) in 1,6 1 THF und läßt abkühlen. Bei 20-22° wird anschließend eine Lösung von 315 g IIa in 1 l THF innerhalb 2 Std. zugetropft. Man rührt noch 1 Std., gießt in gesättigte $NH_4Cl$-Lösung, arbeitet wie üblich auf (Ethylacetat) und erhält 811 g rohes 2α-(2-Hydroxy-7-trimethylsilyloxy-heptyl )-3-cyclopenten-1α-ol.

b) Eine Lösung von 650 g dieser Verbindung in 3,25 1 Dichlormethan wird mit 200 g $CaCO_3$ sowie 3 g Vanadin(IV)oxid-acetylacetonat ("VA") versetzt. Bei 20° werden unter Rühren innerhalb 45 Min. 628 ml 70 %iges wässeriges tert.-Butylhydroperoxid zugetropft; gleichzeitig wird portionsweise weiteres VA zugegeben, insgesamt 45 g. Man rührt noch 5 Std., gießt in gesättigte $Na_2S_2O_3$-Lösung, filtriert, arbeitet wie üblich auf (Dichlormethan) und erhält 685 g rohes 2α-(2-Hydroxy-7-trimethylsilyloxyheptyl)-3α , 4α-epoxy-cyclopentan-1α-ol.

Verwendungsbeispiel

Eine Lösung von 665 g der vorstehenden Verbindung in einem Gemisch von je 5,32 1 Aceton und THF wird bei -2° innerhalb 3,5 Std. tropfenweise mit 2,7 1 Jones-Reagenz (erhalten durch Einrühren von 721 g $CrO_3$ in 621 ml konz. $H_2SO_4$ und Auffüllen mit Wasser) versetzt. Man rührt noch 1 Std. bei 0°, gibt dann unter Kühlung 800 ml Isopropanol hinzu, rührt weitere 30 Min., fügt 1 l Wasser hinzu, trennt ab und extrahiert mit Dichlormethan. Die vereinten organischen Phasen werden mit gesättigter Nacl-Lösung gewaschen, getrocknet und eingedampft. Der Rückstand wird chromatographisch gereinigt. Man erhält 6-Oxo-7- (2α,3α-epoxy-5-oxo-1α-cyclopentyl)-heptansäure ("A"),F. 93-94° .

## Ansprüche

**1.** Cyclopentanderivate der Formel I

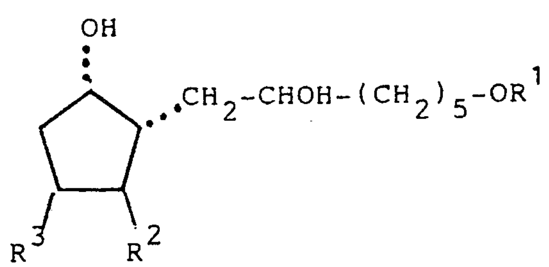

I

worin
$R^1$ H oder eine Hydroxyschutzgruppe und
$R^2$ und $R^3$ zusammen ein O-Atom oder auch zusammen eine Bindung
bedeuten.

**2.** Die Verbindungen der Formel I nach Anspruch 1,
a) 2α-(Hydroxy-7-trimethylsilyloxy-heptyl)-3-cyclopenten-lα-ol;
b) 2α-(2-Hydroxy-7-trimethylsilyloxy-heptyl)-3α, 4α-epoxy-cyclopentan-lα-ol.

**3.** Verfahren zur Herstellung von Verbindungen der Formel I, dadurch gekennzeichnet, daß man ein Lactol der Formel II

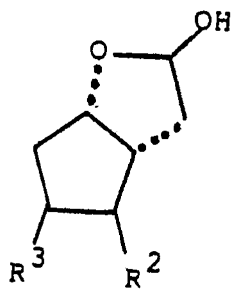

II

worin
$R^2$ und $R^3$ die angegebene Bedeutung haben
mit einer Verbindung der Formel III

$$M-(CH_2)_5-OR^1$$ III

worin
M MgHal oder Li und
Hal Cl, Br oder J bedeuten und
$R^1$ die angegebene Bedeutung hat,
umsetzt
und daß man gegebenenfalls eine erhaltene Verbindung der Formel I ($R^2$ + $R^3$ = Bindung) durch Behandeln mit einem Oxydationsmittel in eine Verbindung der Formel I ($R^2$ + $R^3$ = O) umwandelt.

**4.** Verwendung von Cyclopentanderivaten der Formel I bei der Synthese von Prostaglandinderivaten.

## Claims

**1.** Cyclopentane derivatives of the formula I

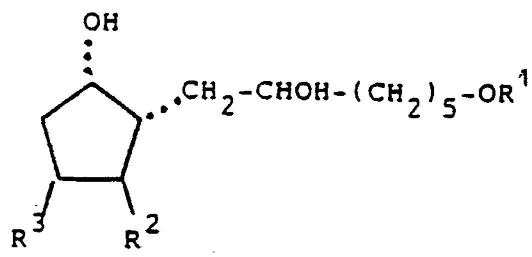

I

in which
$R^1$ is H or a hydroxyl protective group, and
$R^2$ and $R^3$ are together an O atom or are together a bond.

**2.** The compounds of the formula I according to claim 1,
a) 2α-(2-hydroxy-7-trimethylsilyloxyheptyl)-3-cyclopenten-lα-ol;
b) 2α-(2-hydroxy-7-trimethylsilyloxyheptyl)-3α-4α-epoxy-1α-cyclopentanol.

**3.** Process for the preparation of compounds of the formula I, characterised in that a lactol of the formula II

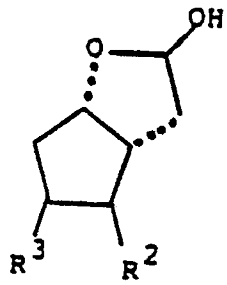

II

in which
$R^2$ and $R^3$ have the above-mentioned meaning, is reacted with a compound of the formula III

$$M-(CH_2)_5-OR^1 \qquad III$$

in which
M is MgHal or Li and
Hal is Cl, Br or I, and
$R^1$ has the above-mentioned meaning,
and in that, where appropriate, a resulting compound of the formula I ($R^2$ + $R^3$ = bond) is converted by treatment with an oxidizing agent into a compound of the formula I ($R^2$ + $R^3$ = 0).

**4.** Use of cyclopentane derivatives of the formula I for the synthesis of prostaglandin derivatives.

## Revendications

**1.** Dérivés du cyclopentane répondant á la formule I

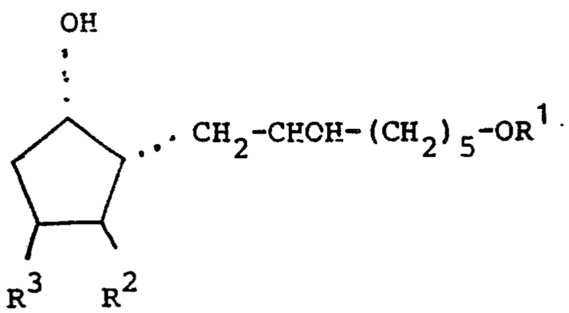

I

dans laquelle
$R^1$ représente n ou un groupe protecteur du groupe hydroxy et
$R^2$ et $R^3$ représentent ensemble un atome d'oxygène ou une liaison.

**2.** Les composés de formule I selon la revendication 1 :
a) 2alpha-(2-hydroxy-7-triméthylsilyloxy-heptyl)-3-cyclopentène-1-alpha-ol;
b) 2alpha-(2-hydroxy-7-triméthylsilyloxy-heptyl)-3alpha, 4alpha-époxy-cyclopentane-1alpha-ol.

**3.** Procédé de préparation des composés de formule I, caractérisé en ce que l'on fait réagir un lactol de formule II.

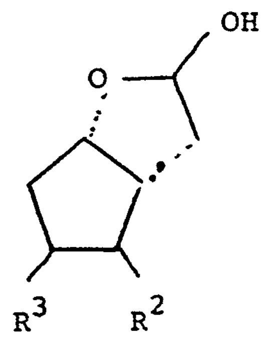

II

dans laquelle
$R^2$ et $R^3$ ont les significations indiquées ci-dessus,
avec un composé de formule III

$$M-(CH_2)_5-OR^1 \qquad III$$

dans laquelle
M représente MgHal ou Li et
Hal représente Cl, Br ou I,
$R^1$ ayant les significations indiquées ci-dessus, ce qui donne un composé de formule I ($R^2 + R^3$ = liaison) qu'on convertit le cas échéant par traitement à l'aide d'un agent oxydant en un composé de formule I ($R^2 + R^3 = 0$).

**4.** Utilisation des dérivés du cyclopentane de formule I pour la synthèse de dérivés de la prostaglandine.